# EUROPEAN PATENT APPLICATION

(11) **EP 1 676 569 A1**
(43) Date of publication of application: **05.07.2006**
(21) Application number: 04031022.9
(22) Date of filing: 30.12.2004
(51) Int. Cl.: A61K 9/19, A61K 9/127

(54) **Lyophilization of virosomes**

(71) Applicant: Pevion Biotech Ltd., 3018 Bern (CH)
(72) Inventor: Zurbriggen, Rinaldo, 3185 Schmitten (CH); Amacker, Mario, 3018 Bern (CH); Rasi, Silvia, 3018 Bern (CH)
(74) Representative: Viering, Jentschura & Partner

(57) **Abstract**

The present invention relates to compositions and methods for the effective lyophilization and reconstitution of virosomes.

## Description

### Field of the invention

The present invention relates to compositions and methods for the effective lyophilization and reconstitution of virosomes.

### Background of the invention

Lyophilization or "freeze-drying" is a technical process for the removal of water. Therein, the aqueous solution is cooled down under its eutectic point, until it is completely frozen. Then the barometric pressure is reduced up to a vacuum, so that the water sublimes and is withdrawn from the solution. The solubilised agent remains as a porous solid, which can later be resolved in water again. The freeze-drying generates solids with a huge surface area, resulting in high water solubility.

Lyophilization is widely used in pharmaceutical applications, as most pharmaceuticals have a limited storage life in solution. Their shelf life can be significantly increased by production of lyophilisates, which are solved, shortly before usage, in an adequate solvent. Although lyophilization has been proven to be a superior preservation technique commonly used today, it has inherent disadvantages. These are mostly coupled to the freezing and reconstitution processes, which are often deleterious for bioactive agents or compositions. To preserve functionality and activity, different techniques have evolved, especially the use of cryoprotectants including for example sugars like sucrose or trehalose.

Liposomes and virosomes have superior properties as drug delivery vehicles. Whereas liposomes are spherical lipid vesicles, virosomes are envelopes of viruses not containing the infectious genetic material of the original virus. The difference of liposomes and virosomes is that virosomes contain additional proteins on their surface making them fusion-active particles, whereas liposomes are inactive carriers.

Thus, virosomes are highly effective adjuvant/carrier systems in modern vaccination, possessing superior properties as antigen delivery vehicles and a strong immunogenic potential whilst concomitantly minimizing the risk of side effects.

To date virosomes have been used effectively in a variety of vaccines. For example, commercially available vaccines against hepatitis A and influenza use virosomes as adjuvants and safe antigen delivery vehicles. Antibodies elicited by the inoculation with antigens reconstituted in virosomes have shown a high affinity for the antigens against which they are raised.

Freeze-drying of liposomes can prevent hydrolysis of the phospholipids and physical degradation of the vesicles during storage. In addition, it may help stabilize the substance that is incorporated in the liposomes. Freeze-drying of a liposome formulation results in a dry cake, which can be reconstituted within seconds to obtain the original dispersion, that is, if the appropriate excipients are used and if suitable freeze-drying conditions are applied. On the other hand the freeze-drying process itself may induce physical changes of the liposomes, such as loss of encapsulated agent and alterations in the vesicle size. The occurrence of such damage is not surprising, because interaction between the hydrophilic phospholipid head groups and water molecules plays a key role in the formation of liposomal bilayers. Thus, removing water from the liposomes by freeze-drying represents an exciting challenge. Moreover, freeze-drying is a time- and energy-consuming process, which certainly requires some expertise in order to avoid its specific pitfalls. Fortunately, excipients, such as disaccharides, have been identified that protect the liposomes during the freezing process (lyoprotectants) and the freeze-drying technique has been extensively described in literature (Pikal et al., 1990, Int. J. Pharm. Sci. 60, 203; Pikal, 1990, Biopharm 10, 18; Essig et al., 1993, "Lyophilization", Wissenschaftliche Verlagsgesellschaft, Stuttgart ; Jenning, 1999, "Lyophilization: Introduction and basic principles", Interpharm Press, Englewood, CO).

Lyoprotectants protect liposomes by (1) preventing fusion of liposomes, (2) preventing the rupture of bilayers by ice crystals, and (3) maintaining the integrity of the bilayers in the absence of water. To do so, the lyoprotectants must form an amorphous glassy matrix in and around the liposomes. Interaction between the lyoprotectant and the phospholipid head groups is considered especially important for preventing leakage during rehydration of liposomes that have a liquid-crystalline bilayer in the hydrated state at ambient temperatures.

It is possible to distinguish different types of liposome formulations with respect to freeze-drying: (1) empty liposomes, which are reconstituted with a solution of the compound to be encapsulated, (2) liposomes loaded with a compound that is strongly associated with the bilayer, and (3) liposomes that contain a water-soluble compound that does not interact with the bilayer. The third one represents the greatest challenge, as both prevention of leakage of encapsulated solutes and preservation of liposome size are required. The bilayer composition is a highly significant factor when determining the resistance of liposomes to freeze-drying stress, but to date it has been difficult to extract general rules from the literature as many other parameters are involved, including lyophilization process conditions, choice of lyoprotectants, and vesicle size.

As depicted above the lyophilization of liposomes has been proven to be demanding at best, but the lyophilization of virosomes is facing even greater problems. This is, in comparison to liposomes, due to the additional proteins in the envelope, responsible for the fusion activity of the virosome. As proteins they are highly susceptible to freeze-drying induced stress causing significant loss of activity.

Thus, there is need for compositions and methods that overcome the problems coupled to the effective lyophilization and reconstitution of fusion-active molecules, namely virosomes.

### Summary of the invention

The present invention provides biologically active compositions and methods for the preparation of highly lyophilization-stress resistant, hydratable virosomal lyophilisates and methods for their reconstitution. According to the invention, biologically active compositions refer to immunogenic compositions or pharmaceutical compositions comprising virosomes and a cationic lipid for effective lyophilisation and reconstitution of the virosome, and, in particular, to an immunogenic composition or a pharmaceutical composition, wherein a cationic lipid for effective lyophilisation and reconstitution of the virosome is present in the membrane of the virosome.

Using the teaching of the invention, virosomes are obtainable which have superior lyophilization and reconstitution properties and which are particularly useful to deliver antigens, drugs and other pharmaceutical active substances including DNA, RNA or siRNA into cells. After lyophilization, they are still capable to deliver said substances to distinct cells through a targeting system, which recognizes surface markers of specific cell types, and, thus are specifically superior to other known delivery vehicles.

In a preferred embodiment, the utilized cationic lipids are cationic cholesteryl derivatives.

In a further embodiment of the invention said cholesterol derivatives are represented by the following formula: 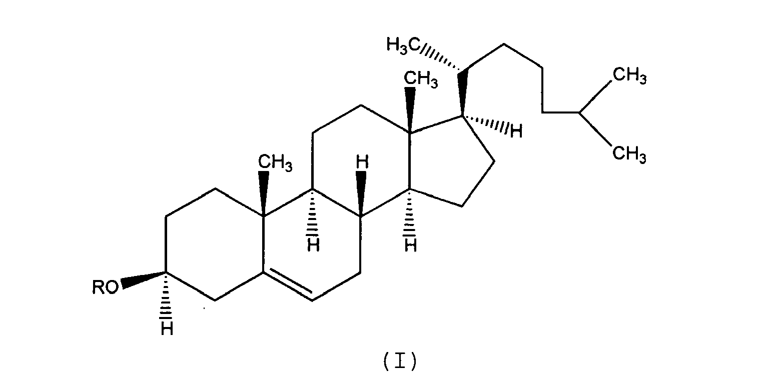 wherein R is selected from the group consisting of R'; R'-(C=O)-; R'-O-(C=O)-; R'-NH-(C=O)-; R'-O-(C=O)-R''-(C=O)-; R'-NH-(C=O)-R''-(C=O)-,
wherein R' is C₁-C₆-alkyl being substituted by at least one positively charged group, preferably a N-containing group of the formula R₁R₂R₃ N⁺- and the respective counter ion is X⁻;
wherein R₁, R₂ and R₃ are independently from each other selected from the group consisting of hydrogen and C₁-C₆-alkyl;
wherein X⁻ is selected from the group consisting of halogen, hydrogen sulphate, sulfonate, dihydrogen phosphate, acetate, trihaloacetate and hydrogen carbonate; and
wherein R'' is C₁-C₆-alkylene.

According to the invention, R'' being C₁-C₆-alkylene stands for a saturated C₁-C₆ alkylene moiety which may be -CH₂-, -(CH₂)₂-, etc which may also be present as branched chain such as - CH(CH₃)-(CH₂)₂- etc.

In a further embodiment, the cholesterol derivatives are represented by the formula II: 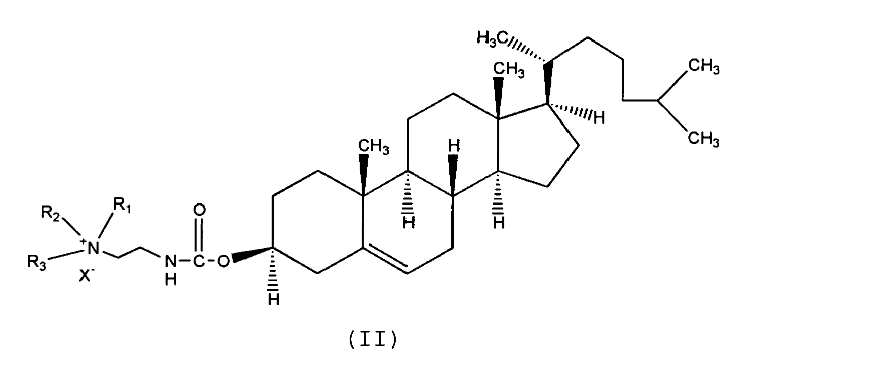 wherein R₁, R₂ and R₃ are independently from each other selected from the group consisting of hydrogen and C₁-C₆-alkyl, and wherein X⁻ is a halogen anion.

In another embodiment, the cationic lipid is represented by formula II, wherein R₁ and R₂ are methyl, R₃ is hydrogen and X⁻ is a halogen anion, preferably chloride, i.e. 3β[N-(N',N'-dimethylammonioethane)-carbamoyl]cholesterol chloride (DC-Chol). In another most preferred embodiment the cationic lipid is represented by formula II, R₁, R₂ and R₃ are methyl and X⁻ is a halogen anion, preferably chloride, i.e. 3β[N-(N',N',N'-trimethylammonioethane)-carbamoyl]cholesterol chloride (TC-Chol).

The virosomes of the invention are fusion-active vehicles delivering a biologically active substance selected from a pharmaceutical agent and an antigenic molecule to a cell. In particular, the virosomes of the invention are antigen-delivery vehicles, capable of eliciting an immune response against a target antigen, or pharmaceutical-delivery vehicles, delivering a pharmaceutical to a cell, and, because of their membrane composition, suitable for lyophilization. In a highly preferred embodiment the virosomes are immunopotentiating reconstituted influenza virosomes (IRIVs).

The virosomal membrane compositions of the present invention comprise preferably between 1.9 and 37 mol% DC-Chol or TC-Chol, relating to the total lipid content of the virosomal membrane. In a highly preferred embodiment, the content of DC-Chol or TC-Chol in the membrane is between 1.9 and 16 mol % of the total lipid content of the virosomal membrane. The residual lipid content of the membrane consists preferably of phospholipids, most preferably phosphatidylcholine and phosphatidylethanolamine in a ratio of 4:1. Additionally, the virosomal membrane may contain an amount of hemagglutinin, sufficient to guarantee fusion activity of the virosome.

In one embodiment of the invention, the composition can additionally contain the desired biologically active substance selected from a pharmaceutical agent and an antigenic molecule in the solution prior to lyophilization. In another embodiment, the pharmaceutical agent or antigen of choice can be added to the lyophilisate prior to the reconstitution process or added after lyophilization in the reconstitution process in combination with the fluid solvent, i.e. solubilized therein.

In further embodiments, the compositions of the invention can further comprise lyoprotectants, such as sucrose, or an adjuvant or adjuvant system.

The invention also comprises methods of lyophilization and reconstitution of the above-mentioned virosomal compositions and the lyophilisate obtained therewith.

In addition, the use of the compositions of the invention for the manufacture of a pharmaceutical for the vaccination and immunization of subject is also intended to be part of the invention. Most preferably the subject is a human being.

Additionally, the present invention also comprises a kit containing the lyophilisates obtainable by using the lyophilization method of the present invention. Furthermore, the kit can additionally comprise a reconstitution solvent and said biologically active substance selected from a pharmaceutical agent and an antigenic molecule, provided that said biologically active substance is not already part of the composition or lyophilisate. In one embodiment, the pharmaceutical agent or antigenic molecule is to be dissolved in the reconstitution solvent prior to reconstitution of the virosome lyophilisate.

The kit provides means to easily prepare an immunogenic composition with a target antigen of choice, e.g. for vaccination, and at the same time provides a prolonged shelf-life and superior storage and handling properties.

In addition, the use of a cationic lipid as described above to enhance the immunogenicity of a virosome is also part of the invention.

### Brief description of figures

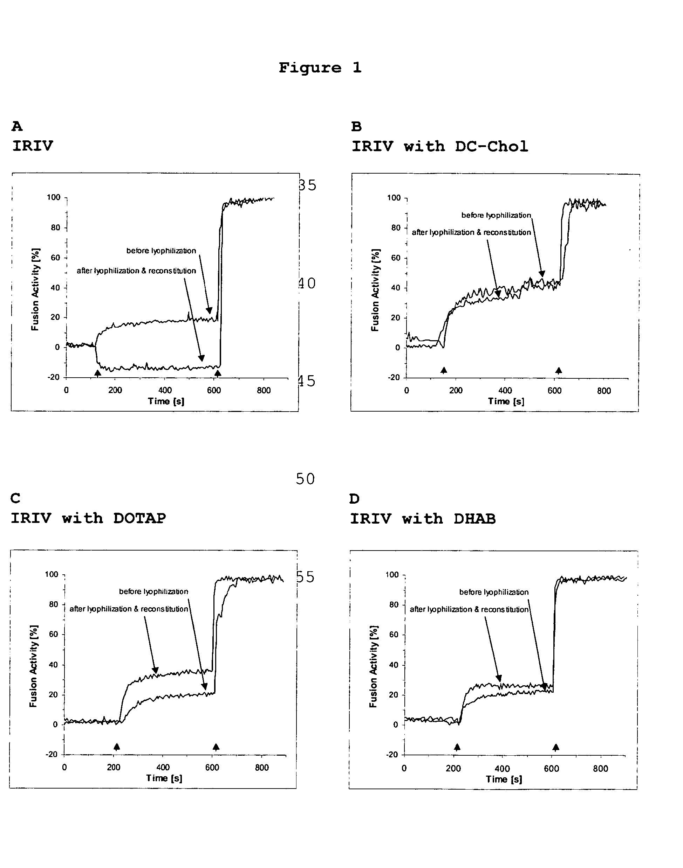

**Figure 1** shows the fusion activity of IRIVs with different membrane compositions before and after lyophilization measured by a FRET assay (see Example 9). Compared are IRIVs without an additional lipid (A), with DC-Chol (B), DOTAP (C) and DHAB (D).

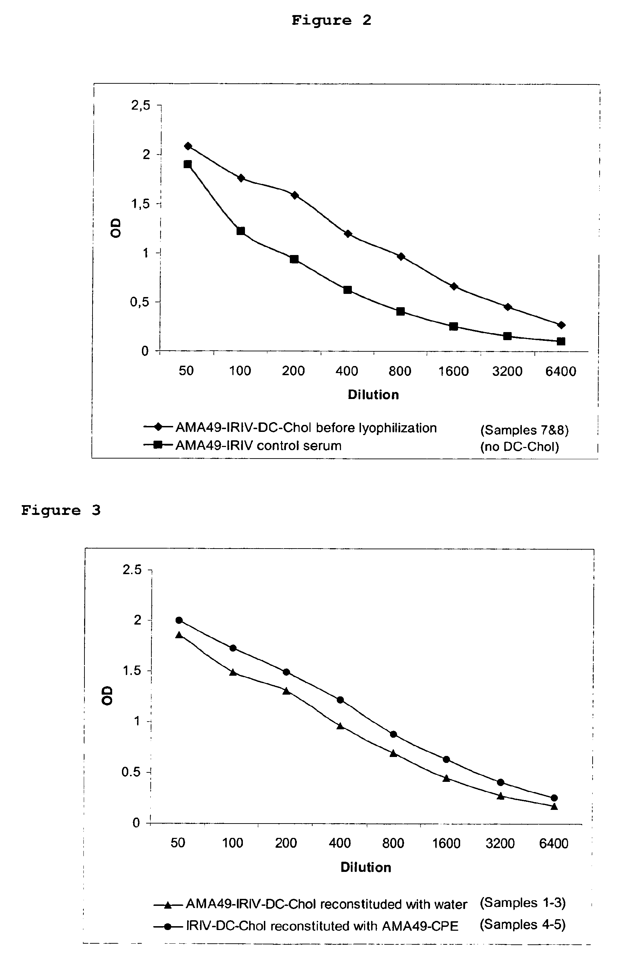

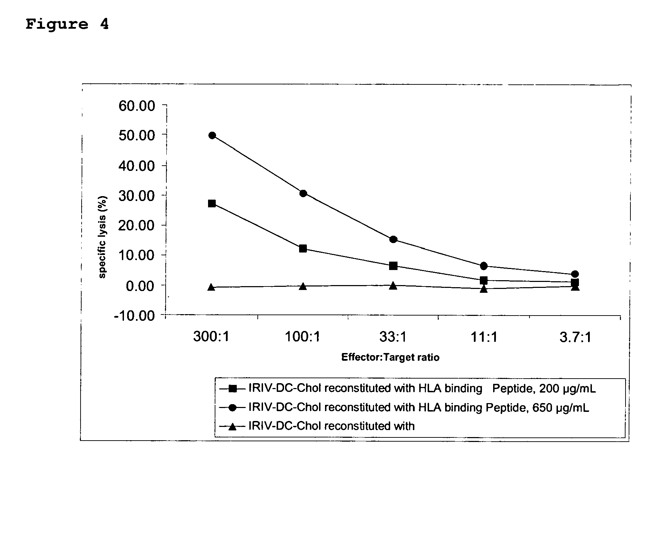

**Figure 2** shows an ELISA (see Example 15) of mice sera immunized with IRIV DC-Chol containing AMA49-CPE peptide on the virosomal surface. Compared are AMA49-IRIV-DC-Chol before lyophilization, AMA49-IRIV-DC-Chol after lyophilization and reconstitution with water, IRIV-DC-Chol after lyophilization and reconstitution with AMA49-CPE peptide and AMA49-IRIV control serum.

**Figure 3** shows a CTL assay (see Example 14) of mice immunized with IRIV-DC-Chol containing HLA-binding peptide within the virosome. Compared are DC-Chol-IRIVs reconstituted with water, 200 µg/ml and 650 µg/ml HLA-binding peptide.

### Detailed description of the invention

The present invention discloses biologically active compositions and methods for the lyophilization and reconstitution of virosomes. To achieve preservation of the fusion-activity of the virosomes of the present invention, special membrane compositions are disclosed herein. These compositions are integral part of the invention and comprise along with phospholipids and the viral protein hemagglutinin cationic lipids, to provide superior freeze-drying stress-resistance for the virosomes of the invention.

### Cationic lipids

The present invention relates to an immunogenic composition comprising virosomes and a cationic lipid for effective lyophilisation and reconstitution of the virosome, and, in particular, to an immunogenic composition, wherein a cationic lipid for effective lyophilisation and reconstitution of the virosome is present in the membrane of the virosome. In a preferred embodiment of the present invention the cationic lipids used as integral membrane components are DOTMA, DOTAP, DPPES, DOGS, DOSPA, DOSPER, THDOB, DOPA, DOTP, DOSC, DOTB, DOPC, DOPE and preferably cholesteryl derivatives.

Preferred cholesterol derivatives are represented by the following formula: 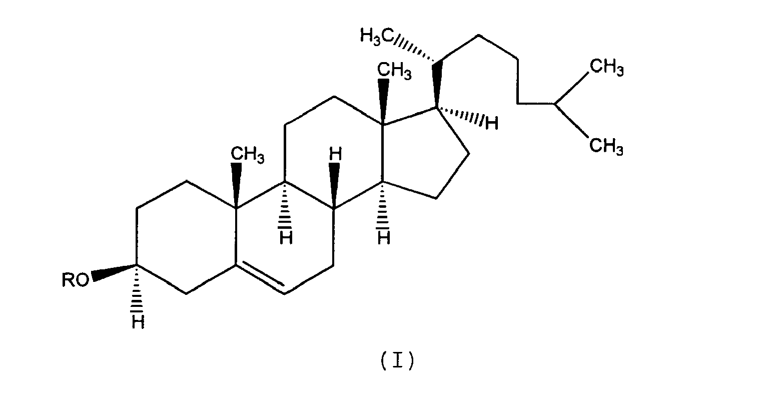 wherein R is selected from the group consisting of R'; R'-(C=O)-; R'-O-(C=O)-; R'-NH-(C=O)-; R'-O-(C=O)-R''-(C=O)-; R'-NH-(C=O)-R''-(C=O)-,
wherein R' is C₁-C₆-alkyl being substituted by at least one positively charged group, preferably a N-containing group of the formula R₁R₂R₃ N⁺- and the respective counter ion is X⁻;
wherein R₁, R₂ and R₃ are independently from each other selected from the group consisting of hydrogen and C₁-C₆-alkyl;
wherein X⁻ is selected from the group consisting of halogen, hydrogen sulphate, sulfonate, dihydrogen phosphate, acetate, trihaloacetate and hydrogen carbonate; and
wherein R'' is C₁-C₆-alkylene.

According to the invention, R'' being C₁-C₆-alkylene stands for a saturated C₁-C₆ alkylene moiety which may be -CH₂-, -(CH₂)₂-, etc which may also be present as branched chain such as - CH (CH₃) - (CH₂) ₂- etc.

In an even more preferred embodiment, the cholesterol derivatives are represented by the formula II: 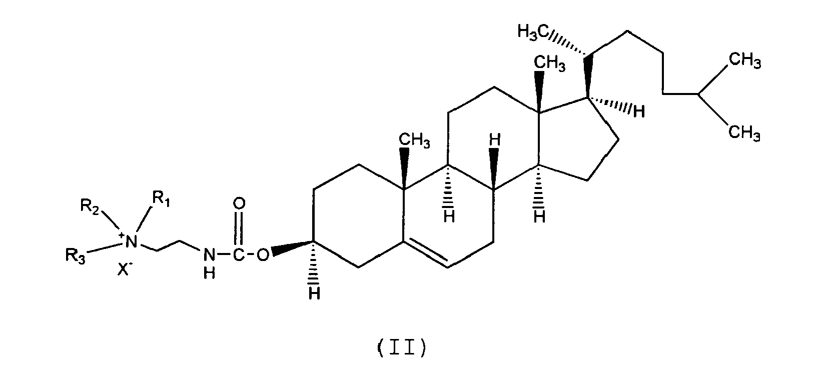 wherein R₁, R₂ and R₃ are independently from each other selected from the group consisting of hydrogen and C₁-C₆-alkyl, and wherein X⁻ is a halogen anion.

In the most preferred embodiment, the cationic lipid is represented by formula II, R₁ and R₂ are methyl, R₃ is hydrogen and X⁻ is a halogen anion, preferably chloride, to yield 3β[N-(N',N'-dimethylammonioethane)-carbamoyl]cholesterol chloride (DC-Chol).

In another most preferred embodiment, the cationic lipid is represented by formula II, R₁, R₂ and R₃ are methyl and X⁻ is a halogen anion, preferably chloride, to yield 3β[N-(N',N',N'-trimethylammonioethane)-carbamoyl]cholesterol chloride (TC-Chol). Both, DC-Chol and TC-Chol, were found to provide superior properties in preserving virosomal fusion-activity after lyophilization and reconstitution.

### Virosomes

Virosomes are envelopes of viruses, and do not contain the infectious genetic material of the original virus. Like liposomes, virosomes can be used to deliver therapeutic substances to a wide variety of cells and tissues, but unlike liposomes, virosomes offer the advantage of efficient entry into the cells followed by the intracellular release of the virosomal content triggered by the viral fusion protein. Moreover, due the incorporation of active viral fusion proteins into their membranes, virosomes release their contents into the cytoplasm immediately after being taken up by the cell, thereby preventing the degradation of the therapeutic substance in the acidic environment of the endosome. Virosomes can further be loaded simultaneously with several different B-cell and T-cell epitopes (Poltl-Frank et al., 1999, Clin. Exp. Immunol. 117:496; Moreno et al., 1993, J. Immunol. 151: 489) including universal T-helper cell epitopes (Kumar et al., 1992, J. Immunol. 148: 1499-1505) and others known to those of skill in the art. Thus, virosomes are highly effective adjuvants in modern vaccination, possessing superior properties as antigen delivery vehicles and a strong immunogenic potential whilst concomitantly minimizing the risk of side effects.

In the present invention, biologically active compositions are disclosed that comprise a biologically active substance selected from a pharmaceutical agent and an antigenic molecule incorporated in synthetic spherical virosomes termed Immunopotentiating Reconstituted Influenza Virosomes (IRIVs). IRIVs are spherical, unilamellar vesicles with a mean diameter of 150 nm and comprise a double lipid membrane, consisting essentially of phospholipids, preferably Phosphatidylcholines (PC) and Phosphatidylethanolamines (PE). In contrast to liposomes, IRIVs contain the functional viral envelope glycoproteins hemagglutinin (HA) and neuraminidase (NA) intercalated in the phospholipid bilayer membrane. The biologically active HA does not only confer structural stability and homogeneity to virosomal formulations but also significantly contributes to the immunological properties by maintaining the fusion activity of a virus.

According to the inventions, said biologically active compositions are capable of delivering biologically active substances to a cellular compartment of an organism. Said biologically active substance is selected from pharmaceutical agents and antigenic molecules, that is preferably selected from the group consisting of DNA, RNA, siRNA, proteins, peptides, amino acids, drugs, pro-drugs and pharmaceutical active substances or derivatives thereof. Preferably the biologically active substance is a pharmaceutical drug, an antigen or a mixture thereof.

Examples for the pharmaceutical agents are selected from the group comprising anaesthetics, angiogenesis inhibitors, anti-acne preparations, anti-allergica, antibiotics and chemotherapeutics for topical use, antihistamines, antiinflammatory/antiinfective, antineoplastic agents, antigens, antiprotozoals, antirheumatics, antiviral vaccines, antivirals, anti-apoptotics, bacterial vaccines, chemotherapeutics, cytostatics, immunosuppressive agents, laxatives and psycholeptics. Preferred examples for the pharmaceutical drug or immuno-active substance are doxorubicin, vinblastine, cisplatin, methotrexate, cyclosporin and ibuprofen.

The term "antigenic molecule" refers to a molecule against which an immune response is desired. This molecule can be selected from a group including, but not limited to, peptides, proteins, lipids, mono-, oligo- and polysaccharides, glycopeptides, carbohydrates, lipopeptides, bacterial or viral pathogens and toxins, other small immunogenic molecules and DNA/RNA coding for such molecules. "Immunogenic" refers to the ability of a molecule to elicit an immune response in an organism inoculated therewith. Examples for antigenic molecules are peptide based T-cell antigens and B-cell antigens. Preferred examples for antigenic molecules are HCV based T-cell antigens, tumor associated antigens, pertussis toxin, cholera toxoid and malaria, RSV and Alzheimer (in particular the beta-amyloid) peptide antigens.

For cancer therapeutic applications of the present invention, any chemotherapeutic drug would be suitable for encapsulation by the virosomes. The methods and compositions of the present invention are further adaptable to any therapeutically relevant application that benefits from the targeted delivery of substances to specific cells and tissues. Such applications may include the targeted delivery of anticancer drugs to cancer cells, antiviral drugs to infected cells and tissues, antimicrobial and anti-inflammatory drugs to affected tissue, as well as the delivery of therapeutics to only those organs and tissues that are affected by the particular disease, thereby increasing the therapeutic index of the therapeutic drug and avoiding systemic toxicity. For example, in tumor therapy, doxorubicin, an anti-tumor antibiotic of the anthracycline class, may be delivered by the methods and compositions of the present invention. Anthracyclines have a wide spectrum of anti-tumor activity and exert pleiotropic effects on the cell. Although they are classic DNA intercalating agents, their mechanism of cytotoxicity is thought to be related to interaction with the enzyme topoisomerase II, production of double-stranded DNA breaks and possibly to the generation of intracellular free radicals that are highly cytotoxic. Thus, the conjugated virosomes can be loaded with doxorubicin in order to selectively and efficiently inhibit tumor progression of established rNeu overexpressing breast tumors.

To date virosomes have been used effectively in a variety of vaccines. For example, commercially available vaccines against hepatitis A and influenza virus. Virosomes have been proven to be excellent and safe adjuvant/carrier systems. Antibodies elicited by the inoculation with antigens reconstituted in virosomes have shown a high affinity for the antigens against which they are raised.

Injected alone most peptide antigens exhibit a relatively low immunogenicity. But in a combined form of antigen and virosome, measurable titers of highly specific antibodies against the antigen can be produced. Antigenic peptides can be delivered via virosomes either on the virosomal surface or encapsulated in the virosome. The difference lies in the type of immune response. When the virosomes fuse with the endosomes after endocytosis, their content, including an encapsulated antigen, is released into the cellular cytoplasm. In the cytoplasm, said content is processed and presented in complex with MHC class I molecules on the cell surface, triggering the cellular, CD8+ cell-mediated, cytotoxic immune response. In contrast to that mechanism, a surface antigen is recognized and endocytosed by B-cells that present it in complex with MHC class II molecules, and, thus, elicit the humoral immune response and the production of specific antibodies.

To increase incorporation rate of biological active substances into virosomes, the handling and to allow longer storage periods, the present invention discloses methods and compositions for the effective lyophilization of the virosomes of the invention. When trying to develop an effective virosome lyophilisate the composition of the bilayer is a crucial factor, which has to be carefully considered. In this context "lyophilisate" refers to the lyophilized composition before reconstitution with a solvent of choice. "Reconstitution" refers to the process of solubilizing the lyophilisate with an appropriate solvent. Therefore, the present invention experimentally addressed the efficiency of different membrane compositions comprising cationic, neutral charged and uncharged lipids to preserve virosomal size and functionality after lyophilization and reconstitution. As a result, the present invention provides virosomal membrane compositions that allow lyophilization and reconstitution of virosomes without loss of function.

Based on these experimental results, the present invention provides highly freeze-drying stress-resistant, hydratable virosomal lyophilisates, comprising cationic cholesteryl derivatives, in particular DC-Chol or TC-Chol as integral membrane components.

Optionally such virosomal lyophilisate additionally comprises a biologically active substance selected from pharmaceutical agents and/or antigenic molecules. These biologically active substances are attached to the virosomal surface or are enclosed therein before lyophilization.

In another embodiment the pharmaceutical agent and/or antigenic molecule is added together with the reconstitution solvent to the virosomal lyophilisate. These pharmaceutical agent and/or antigenic molecule get attached to the newly formed virosomal surface. Preferably the pharmaceutical agent and/or antigenic molecule are conjugated to lipids in order to get attached to the virosomal membrane through lipid. In another embodiment the present invention discloses methods and compositions for efficiently enclose pharmaceutical agents and/or antigenic molecules into the lumen of the newly formed virosomes.

In a preferred embodiment a composition of the present invention comprises 1.9 to 37 mol% of the total lipid content of the virosomal membrane DC-Chol or TC-Chol.

In a most preferred embodiment the DC-Chol or TC-Chol concentration is between 1.9 and 16 mol % of the total lipid content of the virosomal membrane.

The residual lipid content of the virosomal membrane consists of phospholipids, preferably phosphatidylcholine and phosphatidylethanolamine. In a highly preferred embodiment the ratio of phosphatidylcholine and phosphatidylethanolamine contained in the virosomal membrane is 4:1.

All above described compositions comprise a functional amount viral hemagglutinin. In this context "functional amount" refers to an amount sufficient to guarantee fusion-active virosome particles.

A antigenic molecule of choice can be either directly added to one of the compositions described above in an amount sufficient to elicit an immune response, or, solved in the reconstitution buffer, added to the lyophilisate of one of the above-described compositions during the reconstitution process. Thus, the present invention also comprises compositions suitable for lyophilization additionally comprising a target antigen of choice.

The pharmaceutical compound of choice can be either directly added to one of the compositions described above in an amount sufficient to show biological activity, or, solved in the reconstitution buffer, added to the lyophilisate of one of the above-described compositions during the reconstitution process. Thus, the present invention also comprises compositions suitable for lyophilization additionally comprising a target antigen of choice.

The compositions of the present invention can further comprise helper ingredients, which support the lyophilization process. These helper ingredients include, but are not limited to, lyoprotectants as sucrose, trehalose, dextrose, lactose, mannose, xylose and mannitol. Such sugar class compounds are particularly useful in a ratio of 0.1 to 5% in the solution prior to lyophilization. The term "lyoprotectants" refers to a class of compounds useful as helper ingredients during the lyophilization process that are capable of reducing the freeze-drying stress for the virosome.

Also part of the present invention is the method of lyophilizing a composition of the invention basically based on the steps of freezing, primary drying and secondary drying and the following reconstitution process with a solvent or buffer that might optionally contain the desired target antigen.

The use of the disclosed compositions for the manufacture of a pharmaceutical for vaccinating or inoculating a subject therewith is also part of the present invention. Preferably said subject is a human.

Also part of the invention is a kit containing virosomes of the present invention that are already lyophilized. Said kit can, in addition to the virosome lyophilisate, further comprise a reconstitution solvent. Provided that the antigenic molecule is not already part of the lyophilized virosomes said kit can additionally comprise a target antigen. In one embodiment of the invention, the kit contains an antigenic molecule that is to be dissolved in the reconstitution solvent prior to utilizing said reconstitution solvent for solubilizing the lyophilized virosomes.

Additionally, the present invention discloses the use of the above described cationic lipid to further enhance the immunogenicity of the virosome. In this respect the inventors found that the immunogenic properties of the IRIV itself can be further enhanced by the use of a cationic lipid, preferably one of the described cholesterol derivatives, as a virosomal membrane component. In this context the term "immunogenicity" refers to the ability to elicit an immune response.

### Adjuvants

The compositions of the present invention can be further supplemented by combining any of the above-mentioned compositions with a further immune response potentiating compound. Immune response potentiating compounds are classified as either adjuvants or cytokines. Additional adjuvants may further enhance the immunological response by providing a reservoir of antigen (extracellularly or within macrophages), activating macrophages and stimulating specific sets of lymphocytes. Adjuvants of many kinds are well known in the art; specific examples include Freund's (complete and incomplete), mycobacteria such as BCG, M. Vaccae, or Lipid A, or corynebacterium parvum, quil-saponin mixtures such as QS-21 (SmithKline Beecham), MF59 (Chiron), and various oil/water emulsions (e.g. IDEC-AF). Other adjuvants which may be used include, but are not limited to: mineral salts or mineral gels such as aluminium hydroxide, aluminium phosphate, and calcium phosphate; LPS derivates, saponins, surface active substances such as lysolecithin, pluronic polyols, polyanions, peptides or protein fragments, keyhole limpet hemocyanins, and dinitrophenol; immunostimulatory molecules, such as saponins, muramyl dipeptides and tripeptide derivatives, CpG dinucleotides, CpG oligonucleotides, monophosphoryl Lipid A, and polyphosphazenes; particulate and microparticulate adjuvants, such as emulsions, liposomes, virosomes, cochleates; or immune stimulating complex mucosal adjuvants. Cytokines are also useful in vaccination protocols as a result of lymphocyte stimulatory properties. Many cytokines useful for such purposes will be known to one of ordinary skill in the art, including interleukin-2 (IL-2), IL-12, GM-CSF and many others.

### Administration

When administered, the therapeutic compositions of the present invention are administered in pharmaceutically acceptable preparations. Such preparations may routinely contain pharmaceutically acceptable concentrations of salt, buffering agents, preservatives, compatible carriers, supplementary immune potentiating agents such as adjuvants and cytokines and optionally other therapeutic agents. The preparations of the invention are administered in effective amounts. Generally, doses of immunogens ranging from 1 nanogram/kilogram to 100 milligrams/kilogram, depending upon the mode of administration, are considered effective. The preferred range is believed to be between 500 nanograms and 500 micrograms per kilogram. The absolute amount will depend upon a variety of factors, including the composition selected for administration, whether the administration is in single or multiple doses, and individual patient parameters including age, physical condition, size, weight, and the stage of the disease. These factors are well known to those of ordinary skill in the art and can be addressed with no more than routine experimentation.

### Examples

The present invention is illustrated by the following examples, which are not to be construed in any way as imposing limitations upon the scope thereof. On the contrary, it is to be clearly understood that resort may be made to various other embodiments, modifications and equivalents thereof, which after reading the description herein, may suggest themselves to those skilled in the art, but still fall under the scope of the invention.

### Materials and Methods

Chemicals: Octaethyleneglycol-mono-(n-dodecyl)ether (OEG, C₁₂E₈), trifluoroacetic acid (TFA), dihexadecyldimethylammonium bromide (DHAB), L-A-Phosphatidyl-L-Serine from bovine brain (PS), 1,2-Dipalmitoyl-sn-glycero-3-phosphoethanolamine (DPPE), 1,2-Dilauroyl-sn-glycero-3-phosphocholine (DLPC), cholesterol from lanolin, 1-myristoyl-sn-glycero-3-phosphocholine (Lyso-PC), palmitoyl-DL-carnitine chloride and Cholesteryl N-(trimethylammonioethyl)carbamate chloride (TC-Chol) were from Fluka or Sigma (Buchs, Switzerland). Egg phosphatidyl choline (PC) was obtained from Lipoid (Cham, Switzerland). 1,2-Dipalmitoyl-sn-glycero-3-phospho-rac-(1-glycerol) sodium salt (DPPG), 3β-[N-(N',N'-Dimethylaminoethane)-carbamoyl]Cholesterol Hydrochloride (DC-Chol), 1,2-Dipalmitoyl-sn-Glycero-3-Phosphate Monosodium Salt (DPPA) and 1,2-Dipalmitoyl Ethylene Glycol (DPEG) were purchased from Avanti Polar Lipids (Alabaster, AL, USA). 1-Palmitoyl-3 oleoyl-sn- glycero-2-phosphoryl-ethanolamine (PE) was obtained from R. Berchtold (Biochemical Laboratory, University of Bern, Switzerland). Bio-beads SM2 and Bio-Gel A-15m were from BioRad Laboratories (Glattbrugg, Switzerland). Lissamine™ rhodamine B 1,2-dihexadecanoyl-sn-glycero-3-phosphoethanolamine, triethylammonium salt (Rh-DHPE), N-(4,4-difluoro-5,7-diphenyl-4-bora-3a,4a-diaza-s-indacene-3-propionyl)-1,2-dihexadecanoyl-sn-glycero-3-phosphoethanolamine (Bodipy 530/550-DHPE) were from Molecular Probes Europe (Leiden, The Netherlands). N-[1-(2,3-Dioleoyloxy)propyl]-N,N,N-trimethylammonium chloride (DOTAP) was purchased from Roche Applied Science (Rotkreuz, Switzerland). Doxorubicin·HCl is available from Fluka (Buchs, Switzerland).
Viruses: Influenza viruses of the X-31 strain and the A/Sing (A/Singapore/6/86) strain, propagated in the allantoic cavity of embryonated eggs (Gerhard, J. Exp. Med. 144:985-995, 1976), were obtained from Berna Biotech AG (Bern, Switzerland).
Peptides: The HLA-A2.1-binding hepatitis C virus (HCV) HLA-binding peptide (DLMGYIPLV, aa 132-140) (Cerny *et al*., J. Clin. Invest. 95(2):521-30, 1995) as well as an HLA-A2.1-binding control peptide and the malaria mimetic AMA49-CPE ((1,3-Dipalmitoyl-glycero-2-phosphoethanolamino)-Suc-GGCYKDEIKKEIERESKRIKLNDNDDEGNKKIIAPRIFISDDKDSLKCG (Disulfide bond)) (Moreno et al., Chembiochem 2:838-43, 2001) were obtained from Bachem AG (Bubendorf, Switzerland).
Mice: Immunisation experiments were performed two times in independent laboratories in HHD mice transgenic for HLA-A2.1 (A0201) monochain histocompatibility class I molecule and deficient for both H-2D^{b} and murine β2-microglobulin (Pascolo *et al*., J. Exp. Med. 185(12):2043-51,1997). Mice were housed in appropriate animal care facilities and handled according to international guidelines.

### Example 1

Preparation of immunopotentiating reconstituted influenza virosomes (IRIV): Virosomes were prepared by the method described previously (Bron *et al*., Methods Enzymol. 220:313-331, 1993; Zurbriggen *et al*., Prog Lipid Res 39(1):3-18, 2000). Briefly, 32 mg (41.7 µmol) egg PC and 8 mg (11.1 µmol) PE were dissolved in 2 ml of PBS, 100 mM OEG (PBS/OEG). 4 mg HA of influenza virus was centrifuged at 100,000 x g for 1 h at 4°C and the pellet was dissolved in 2 ml of PBS/OEG. The detergent solubilized phospholipids and viruses were mixed and sonicated for 1 min. This mixture was centrifuged at 100,000 x g for 1 h at 20°C and the supernatant was sterile filtered (0.22 µm). Virosomes were then formed by detergent removal using 180 mg of wet SM2 Bio-Beads for 1 h at room temperature with shaking and three times for 30 min with 90 mg of SM2 Bio-Beads each. The final concentrations of lipids were 8 mg/ml (10.4 µmol/ml) PC and 2 mg/ml (2.7 µmol/ml) PE.

The hemagglutinin/phospholipid ratio was determined by phospholipid determination after Böttcher (Böttcher *et al*., Anal. Chim. Acta 24:202-203, 1961) and HA-quantification after SDS-PAGE with the Coomassie-extraction method after Ball (Ball, Anal. Biochem. 155:23-27, 1986).

### Example 2

Preparation of immunopotentiating reconstituted influenza virosomes containing DC-Chol (DIRIV): Virosomes were prepared by the method described previously (Bron *et al*., Methods Enzymol. 220:313-331, 1993; Zurbriggen *et al*., Prog. Lipid Res. 39(1):3-18, 2000). Briefly, 32 mg (41.7 µmol) egg PC, 8 mg (11.1 µmol) PE and 0.3 - 5 mg (0.6-10 µmol) DC-Chol were dissolved in 2 ml of PBS, 100 mM OEG (OEG-PBS). 4 mg HA of influenza virus was centrifuged at 100,000 x g for 1 h at 4°C and the pellet was dissolved in 1 ml of PBS/OEG. The detergent solubilized phospholipids and viruses and 1 ml of 20% (w/v) sucrose were mixed to a final volume of 4 ml and sonicated for 1 min. This mixture was centrifuged at 100,000 x g for 1 h at 20°C and the supernatant was sterile filtered (0.22 µm). Virosomes were then formed by detergent removal using 180 mg of wet SM2 Bio-Beads for 1 h at room temperature with shaking and three times for 30 min with 90 mg of SM2 Bio-Beads each. The final concentrations of lipids were 8 mg/ml (10.4 µmol/ml) PC, 2 mg/ml (2.7 µmol/ml) PE and 0.075-1.25 mg/ml (0.12-2.5 µmol/ml) DC-Chol.

The hemagglutinin/phospholipid ratio was determined by phospholipid determination after Böttcher (Böttcher *et al*., Anal. Chim. Acta 24:202-203, 1961) and HA-quantification after SDS-PAGE with the Coomassie-extraction method after Ball (Ball, Anal. Biochem. 155:23-27, 1986).

### Example 3

Preparation of AMA49-DIRIV: Method of constructing DIRIV with lipid bound antigen: The preparation of virosomes wherein the antigens are attached to the virosome surface. For the preparation of PE-mimetic-IRIV, a solution of purified Influenza A/Singapore hemagglutinin (4 mg) in phosphate buffered saline (PBS) was centrifuged for 30 min at 100 000g and the pellet was dissolved in PBS (1.33 ml) containing 100 mM octaethyleneglycolmonodecylether (PBS-OEG). AMA49-PE conjugates (4 mg), phosphatidylcholine (32 mg; Lipoid, Ludwigshafen, Germany) and phosphatidylethanolamine (6 mg) were dissolved in a total volume of 2.66 ml of PBS-OEG. The phospholipid and the hemagglutinin solutions were mixed and sonicated for 1 min. This solution was then centrifuged for 1 hour at 100 000g and the supernatant was filtered (0.22 µm) under sterile conditions. Virosomes were then formed by detergent removal using BioRad SM BioBeads (BioRad, Glattbrugg, Switzerland). DIRIV were stored in aliquots at - 70°C before lyophilization.

### Example 4

### Method of constructing DIRIV with targeting ligand and spacer:

This example demonstrates the site-directed conjugation of the Fab' fragment to the flexible spacer arm designed to keep the antigen binding site available for binding to the target cell. In order to place the Fab' molecules in a position which allows their bivalent binding potential to remain available, Fab'-fragments are conjugated to the flexible spacer arm by site-directed conjugation. 100 mg of NHS-PEG-MAL containing a long polyethylene glycol spacer arm (PEG) are dissolved in 3 ml of anhydrous methanol containing 10 µl of triethylamine. Then, 45 mg of dioleoyl phosphatidylethanolamine dissolved in 4 ml of chloroform and methanol (1:3;v/v) are added to the solution. The reaction is carried out under nitrogen for 3h at room temperature (RT). Methanol/chloroform is removed under decreasing pressure and the products are redissolved in chloroform. The solution is extracted with 1% NaCl to remove unreacted material and water-soluble by-products. The PE-PEG-MAL is further purified by silic acid chromatography as described by Martin et al. (1982), with some modifications:
the silica gel column has a diameter of 1.5 cm and is loaded with 14 silica gel (Kieselgel 60, Fluka 60752). Elution is performed with the following gradient: Chloroform:methanol 29:1, 28:2, 27:3, 26:4 (ml) etc. 6 ml fractions are collected. PEG-PEG-MAL is obtained in fractions 13-31. Fractions and purity of PE-PEG-MAL are analyzed by TLC on silicon with chloroform-methanol-water 65:25:4. PE-PEG-MAL is dissolved in Tris-HCl buffer (100mM, pH 7.6) containing 10mg/150 µl of octaethylenglycol-monododecylether (C₁₂E₈). To this solution the Fab'-fragments are added at a Fab'/PE-PEG-MAL ratio of 1:10. The solution is stirred at RT for 2 hr under nitrogen. Further C₁₂E₈ is added to obtain a 1%-C₁₂E₈₋solution and the reaction mixture is stirred overnight at 4°C. Unreacted PE-PEG-MAL is removed by the addition of 400 µl of washed, moist Thiopropyl Sepharose 6B. After a 3-hour incubation, the gel is removed by centrifugation. PE-PEG-Fab' -solution (3.6 ml) is sterilized by passage through a 0.2-µm filter and stored as a 0.01 M C₁₂E₈ detergent solution.

### Example 5

Method of Producing FAB' DIRIV: This example demonstrates the preparation of conjugated virosomes targeted to specific cells. Hemagglutinin (HA) from the A/Singapore/6/86 strain of influenza virus is isolated as described in Waelti and Glueck, Int. J. Cancer 77: 728-733, 1998. Supernatant containing solubilized HA trimer (2.5 mg/ml) in 0-01M C₁₂E₈ detergent solution is used for the production of virosomes. Phosphatidylcholine (38mg) in chloroform is added to a roundbottom flask and the chloroform evaporated by a rotary evaporator to obtain a thin PC (phosphatidylcholine) film on the glass wall. The supernatant (4 ml containing 10 mg HA) and 3.6 ml of PE-PEG-Fab' (containing 4 mg Fab'-fragments) from Example 3 are added to this flask. Under gentle shaking, the PC film covering the glass wall of the flask is solubilized by the C12E8 detergent containing mixture. The detergent of the resulting solution is removed by extraction with sterile Biobeads SM-2. The container is shaken for 1 hr by a REAX2 shaker (Heidolph, Kelheim, Germany). To remove the detergent completely, this procedure is repeated three times with 0.58 mg of Biobeads, after which a slightly transparent solution of Fab'-Virosomes is obtained. Quantitative analysis reveals that 1 ml of Fab'-Virosomes contain 1.3 mg of HA, 5 mg of PC and 0.53 mg of Fab'-fragments. Concentrations of Fab' are determined by an immunoassay of the fractions collected from the gel filtration on the High Load Superdex 200 column as described in Antibodies, A Laboratory Manual. The procedure for the production of virosomes without Fab' is the same except that no PE-PEG-Fab' is added.

### Preparation of immunopotentiating reconstituted influenza virosomes containing DC-Chol (DIRIV) and bearing PE-PEG-Fab':

Virosomes were prepared by the method described previously (Bron et al., Methods Enzymol. 220:313-331, 1993; Zurbriggen et al., Prog. Lipid Res. 39(1):3-18, 2000). Briefly, 32 mg (41.7 µmol) egg PC, 8 mg (11.1 µmol) PE and 0.3 - 5 mg (0.6-10 µmol) DC-Chol and the previously formed PE-PEG-Fab' were dissolved in 2 ml of PBS, 100 mM OEG (OEG-PBS). 4 mg HA of influenza virus was centrifuged at 100,000 x g for 1 h at 4°C and the pellet was dissolved in 1 ml of PBS/OEG. The detergent solubilized phospholipids and viruses and 1 ml of 20% (w/v) sucrose were mixed to a final volume of 4 ml and sonicated for 1 min. This mixture was centrifuged at 100,000 x g for 1 h at 20°C and the supernatant was sterile filtered (0.22 µm). Virosomes were then formed by detergent removal using 180 mg of wet SM2 Bio-Beads for 1 h at room temperature with shaking and three times for 30 min with 90 mg of SM2 Bio-Beads each. The final concentrations of lipids were 8 mg/ml (10.4 µmol/ml) PC, 2 mg/ml (2.7 µmol/ml) PE and 0.075-1.25 mg/ml (0.12-2.5 µmol/ml) DC-Chol.

The hemagglutinin/phospholipid ratio was determined by phospholipid determination after Bbttcher (Böttcher et al., Anal. Chim. Acta 24:202-203, 1961) and HA-quantification after SDS-PAGE with the Coomassie-extraction method after Ball (Ball, Anal. Biochem. 155:23-27, 1986).

### Example 6

### Loading DIRIV with a pharmaceutical composition of interest:

Doxorubicin is loaded into virosomes through a proton gradient generated by virosome-entrapped ammonium sulfate as described by Gabizon et al., J. Natl. Cancer Inst. 81: 1484-1488, 1989. To load virosomes with ammonium sulfate, an ammonium sulfate solution (4.17 g/ml) is added to the DIRIV solution (7.5 ml), sonicated for 1 min and dialysed (Spectra/Por 2.1, Biotech DispoDialyzers, MWCO: 15'000, Spectrum Medical Industries, Houston, TX, USA) against 1 liter of PBS containing 5% of glucose for 24 hours at 4 °C. After 24 hours the dialysis buffer is changed and the virosome solution dialyzed for a further 24 hours. To prepare the doxorubicin loading solution, 10 mg of doxorubicin is dissolved in 3 ml of water and sterilized through a 0.2-µm filter, then 750 µl of sterile 5X concentrated PBS and 5% glucose are added.

The virosome solution and doxorubicin loading solution are warmed to 33° C, and then 2 volumes of virosome solution are mixed with 1 volume of doxorubicin loading solution. The mixture is incubated for 10 h at 33°C and further incubated overnight at 28°C. Non-encapsulated doxorubicin is separated from the virosomes by gel filtration on a High Load Superdex 200 column (Pharmacia, Uppsala, Sweden), equilibrated with sterile PBS, 5% glucose. The void volume fractions containing Fab'-virosomes with encapsulated doxorubicin are eluted with 5% glucose in PBS and collected.

### Example 7

Lyophilization of DIRIV: DIRIV were stored in aliquots at - 70°C before lyophilization. Lyophilization was done in a Savant AES1010 speedvac according to the supplier's instructions. Dried samples were used immediately or stored at -70°C. For reconstitution of lyophilized DIRIV, a volume of water equal to the volume before lyophilization was added to the dried DIRIV. Reconstituted empty DIRIV were stored at 4°C.

### Example 8

Preparation of HLA-binding Peptide-DIRIV: DIRIV were stored in aliquots at -70°C before lyophilization. Lyophilization was done in a Savant AES1010 speedvac according to the supplier's instructions. Dried samples were used immediately or stored at -70°C. For reconstitution of lyophilized DIRIV, a volume of HLA-binding peptide dissolved in water equal to the volume before lyophilization was added to the dried DIRIV. Reconstituted HLA-binding Peptide-DIRIVs were stored at 4°C. Determination of encapsulated peptide concentration was done by RP-HPLC.

### Example 9

DIRIV were stored in aliquots at -70°C before lyophilization. Lyophilization was done in a Savant AES1010 speedvac according to the supplier's instructions. Dried samples were used immediately or stored at -70°C. For reconstitution of lyophilized DIRIV, a volume of AMA49-CPE dissolved in water equal to the volume before lyophilization was added to the dried DIRIV. Reconstituted AMA49-DIRIVs were stored at 4°C. Determination of incorporated peptide concentration was done by RP-HPLC.

### Example 10

Preparation of DOXRUBICINE-DIRIV: DIRIV were stored in aliquots at -70°C before lyophilization. Lyophilization was done in a Savant AES1010 speedvac according to the supplier's instructions. Dried samples were used immediately or stored at -70°C. To prepare the doxorubicin loading solution, 10 mg of doxorubicin is dissolved in 3 ml of water and sterilized through a 0.2-µm filter. For reconstitution of lyophilized DIRIV, a volume of DOXRUBICINE equal to the volume before lyophilization was added to the dried DIRIV. Reconstituted DOXRUBICINE-DIRIVs were stored at 4°C.

### Example 11

Determination of incorporated DOXRUBICINE: The amount of encapsulated drug, in this case, doxorubicin, is determined by absorbance at 480 nm. DIRIV preparations contain on average 150 µg/ml doxorubicin. The mean diameter of the virosomes is determined by photon-correlation spectroscopy (PCS) with a Coulter N4Plus Sub-Micron-Particle Size Analyzer (Miami, FL, USA). The proper expression of viral fusogenic activity of the virosomes is measured as previously described by Hoekstra et al., Biochemistry 23: 5675-5681, 1984, by an assay based on octadecylrhodamine (R18) fluorescence dequenching.

### Example 12

Preparation of immunopotentiating reconstituted influenza virosomes containing other lipids: Virosomes were prepared as described in example 3 with the only difference that DC-Chol was replaced by one of the following substances: DHAB, DOTAP, PS, cholesterol, DPPE, DLPC, Lyso-PC, palmitoyl-DL-carnitine, DPEG or TC-Chol. The final concentrations of lipids were 8 mg/ml (10.4 µmol/ml) PC, 2 mg/ml (2.7 µmol/ml) PE and 0.125 mg/ml (0.22 µmol/ml) DHAB, or 0.125 mg/ml (0.18 µmol/ml) DOTAP, or 2-8 mg/ml (2.8-11.3 µmol/ml) PS, or 0.125 mg/ml (0.32 µmol/ml) cholesterol, or 0.125 mg/ml (0.18 µmol/ml) DPPE, or 0.125 mg/ml (0.19 µmol/ml) DLPC, or 0.125 mg/ml (0.27 µmol/ml) Lyso-PC, or 0.125 mg/ml (0.29 µmol/ml) palmitoyl-DL-carnitine, or 0.135 mg/ml (0.25 µmol/ml) DPEG, or 0.125 mg/ml (0.23 µmol/ml) TC-Chol, respectively.

Modified IRIV were stored in aliquots at -70°C before lyophilization. Lyophilization was done in a Savant AES1010 speedvac according to the supplier's instructions. Dried samples were used immediately or stored at -70°C. For reconstitution of lyophilized virosome, a volume of water or HLA-binding Peptide PBS in water equal to the volume before lyophilization was added to the dried virosome. Reconstituted virosomes were stored at 4°C.

### Example 13

HLA-binding peptide quantification: Peptide quantification was done by HPLC on an Agilent 1100 Series (Agilent Technologies, Switzerland) using a CC 125/4.6 Nucleosil 100-5 C8 reversed-phase column (Macherey-Nagel, Switzerland) (RP-HPLC). The following eluents were used: buffer A, 10 mM TEAP in water; buffer B, 100 % acetonitrile. HPLC program: flow rate 1.3 ml/min; buffer and column temperature 25°C; buffer starting concentration: 25% B; 0-7 min: increase of buffer B to 38%; 7-12.4 min: increase of buffer B to 100%; 12.4-16.4 min: 100% buffer B. For quantification of encapsulated peptide, a fraction (5-30 µl) of virosomes were loaded on freshly prepared, PBS-equilibrated 1 ml Sephadex G50 Coarse gel-filtration spin columns. Vesicles with encapsulated peptide only were obtained after centrifugation of the spin column at 300 x g for 2 min, as the non-encapsulated peptide was retarded in the column.

### Example 14

AMA49-CPE peptide quantification: Peptide quantification was done by HPLC on an Agilent 1100 Series (Agilent Technologies, Switzerland) using a ZORBAX Eclipse XDB-C8 reversed-phase column (Agilent Technologies, Switzerland) (RP-HPLC). The following eluents were used: buffer A, 0.1% TFA in water; buffer B, 0.1% TFA in methanol. HPLC program: flow rate 1.0 ml/min; buffer and column temperature 60°C; buffer starting concentration: 60% B; 0-15 min: increase of buffer B to 100%; 15-20 min: 100% buffer B. For quantification of encapsulated peptide, a fraction (5-30 µl) of virosomes were loaded on freshly prepared, PBS-equilibrated 1 ml Sephadex G50 Coarse gel-filtration spin columns. Vesicles with encapsulated peptide only were obtained after centrifugation of the spin column at 300 x g for 2 min, as the non-encapsulated peptide was retarded in the column.

### Example 15

FRET Assay: For *in vitro* fusion measurements by fluorescence resonance energy transfer (FRET) (Struck et al., Biochemistry 20(14):4093-99, 1981; Loyter *et al*., Methods Biochem. Anal. 33:129-64, 1988), the following assay was developed: 0.75 mol% of Bodipy 530/550-DHPE and 0.25 mol% of N-Rh-DHPE were incorporated into liposomes consisting of PC/DPPG (70:30). Fluorescence measurements were carried out at discrete temperatures between 4°C and 42°C in 5 mM sodium phosphate buffer pH 7.5, 100 mM NaCl, in a final volume of 0.8 ml in 2.5 ml PMMA micro-cuvettes (VWR, Switzerland) under continuous stirring. Typically, 1 µl of labelled liposomes (0.3 nmol phospholipid) were mixed with 5-20 µl of virosomes (0.1-0.4 nmol phospholipid) and fusion was triggered by addition of 3.75-7 µl of 1 M HCl, resulting in a pH of 4.5. The increase in fluorescence was recorded every 5 seconds at excitation and emission wavelengths of 538 nm and 558 nm, respectively, with an excitation slit of 2.5 nm and an emission slit of 15.0 nm. Measurements were carried out with an LS 55 Luminescence spectrometer (Perkin Elmer Instruments, USA) equipped with a thermostated cuvette holder and a magnetic stirring device. The maximal fluorescence at infinite probe dilution was reached after addition of Triton X-100 (0.5% v/v final concentration). For calibration of the fluorescence scale the initial residual fluorescence of the liposomes was set to zero and the fluorescence at infinite probe dilution to 100%.

### Example 16

Particle size determination was done by light scattering using a Zetasizer 1000HS instrument (Malvern Instruments, UK) in 2 ml PMMA cuvettes (Sarstedt AG, Switzerland). 5-20 µL of virosomes or liposomes, respectively, were diluted in filtered (0.22 µm) PBS and measured three times for 300 sec at 25°C and 633 nm according to the supplier's instructions.

### Example 17

Preparation of liposomes containing DC-Chol (DC-liposomes): 32 mg (41.7 µmol) PC, 8 mg (11.1 µmol) PE and 0.8-5 mg (1.6-10 µmol) DC-Chol were dissolved in 4 ml of PBS, 100 mM OEG, 5% (w/v) sucrose (OEG-PBS), then mixed and sonicated for 1 min. This mixture was sterile filtered (0.22 µm) and liposomes were then formed by detergent removal using 180 mg of wet SM2 Bio-Beads for 1 h at room temperature with shaking and three times for 30 min with 90 mg of SM2 Bio-Beads each. The final concentrations of lipids were 8 mg/ml PC (10.4 µmol/ml), 2 mg/ml PE (2.7 µmol/ml) and 0.2-1.25 mg/ml DC-Chol (0.4-2.5 µmol/ml). Liposomes were stored in aliquots at -70°C before lyophilization. Lyophilization was done in a Savant AES1010 speedvac according to the supplier's instructions. Dried samples were used immediately or stored at -70°C. For reconstitution of lyophilized DC-liposomes, water or HLA-binding peptide dissolved in water, respectively, was added to the dried DC-liposomes. Reconstituted HLA-binding Peptide-DC-liposomes were stored at 4°C.

### Example 18

Preparation of liposomes: 78 mg (101.6 µmol) PC (dissolved in methanol) and 32.68 mg (43.56 µmol) DPPG (dissolved in methanol/chloroform (1:1)) (molar ratio 70:30) were mixed together and the solvent was removed by using a rotary evaporator (Rotavapor R-205, Büchi Labortechnik, Switzerland) at 40°C at a gradual vacuum of 30-10 kPa. The dried lipid film was rehydrated with 1.5 ml 5% (w/v) sucrose in water. Liposomes were stored in aliquots at -70°C before lyophilization. Lyophilization was done in a Savant AES1010 speedvac according to the supplier's instructions. Dried samples were used immediately or stored at -70°C. For reconstitution of lyophilized liposomes, PBS or HLA-binding peptide dissolved in PBS, respectively, was added to the dried liposomes. Reconstituted HLA-binding Peptide-liposomes were stored at 4°C.

### Example 19

Preparation of liposomes containing DC-Chol (DC-liposomes): 66.8-75.2 mg (87.1-98 µmol) PC (dissolved in methanol) and 32.68 mg (43.56 µmol) DPPG (dissolved in methanol/chloroform (1:1)) and 1.82-7.26 mg (3.6-14.5 µmol) DC-Chol (dissolved in methanol) (molar ratio 60-67.5:30:2.5-10) were mixed together and the solvent was removed by using a rotary evaporator (Rotavapor R-205, Büchi Labortechnik, Switzerland) at 40°C at a gradual vacuum of 30-10 kPa. The dried lipid film was rehydrated with 1.0 ml 5% (w/v) sucrose in water. Liposomes were stored in aliquots at -70°C before lyophilization. Lyophilization was done in a Savant AES1010 speedvac according to the supplier's instructions. Dried samples were used immediately or stored at -70°C. For reconstitution of lyophilized DC-liposomes, PBS or HCV HLA-binding peptide dissolved in PBS, respectively, was added to the dried DC-liposomes. Reconstituted HLA-binding Peptide-DC-liposomes were stored at 4°C.

### Example 20

Immunisation and cytotoxicity assay: HLA-2.1 tg mice were immunised subcutaneously (*sc*.) at the base of the tail with 100 µl of the corresponding virosome formulation. Mice received 2 injections at a 3-week interval and the response was analysed 2 weeks after the last injection. Spleen cells (4 x 10⁶/well) from immunised mice were restimulated for 5 days in 24-well tissue culture plates with 2 x 10⁶ irradiated (1500 rad) spleen cells that have been pulsed with 10 µg/ml peptide, in complete RPMI medium (Sigma Aldrich, St. Louis, MO) containing 2 mM L-Glutamine, 100 U penicillin, 100 µg/ml Streptomycin (Sigma Aldrich), 5 mM Hepes, 10% FCS (Gibco BRL, Basel, Switzerland) and 5 x 10⁻⁵ M 2-mercaptoethanol at 37°C and 5% CO₂. On day 2, 5 U/ml IL-2 (EuroCetus B.V., Amsterdam, The Netherlands) were added. Specific cytolytic activity was tested in a standard 51Cr release assay against an EL-4S3⁻-Rob HHD target cells pulsed with 10 µg/ml of the selected peptides or medium control. After 4 hr incubation, ⁵¹Cr release was measured by using a γ-counter. Spontaneous and maximum release was determined from wells containing medium alone or after lysis with 1M HCl, respectively. Lysis was calculated by the formula: (release in assay - spontaneous release)/(maximum release - spontaneous release) x 100. Peptide-specific lysis was determined as the percentage of lysis obtained in the presence or in the absence of peptide. Spontaneous release was always less than 15% of maximum release.

### Example 21

### Enzyme-linked immunosorbent assay ELISA against AMA49-CPE:

ELISA microtiter plates (PolySorb, Nunc, VWR International AG, Switzerland) were coated at 4°C overnight with 100 µL/well of 10 µg/ml AMA49-CPE in PBS. Wells were washed three times with 300 µl/well of PBS containing 0.05% Tween-20 before they were blocked with 5% milk powder in PBS for 2h at 37°C. Wells were washed three times with 300 µl/well of PBS containing 0.05% Tween-20. Plates were then incubated with two-fold serial dilutions of mouse serum in PBS containing 0.05% Tween-20 and 0.5% milk powder (100 µl/well) for 2h at 37°C. After washing, the plates were incubated with an alkaline phosphatase conjugated goat anti-mouse IgG (γ-chain specific) antibody (Sigma, St. Louis, MO, USA) for 1h at 37°C and then washed three times. Phosphatase substrate (1 mg/ml p-nitrophenyl phosphate (Sigma) in 0.14% (w/v) Na₂CO₃, 0.3% (w/v) NaHCO₃, 0.02% (w/v) MgCl₂, pH 9.6) was added and incubated at room temperature in the dark. After an appropriate time the reaction was stopped by the addition of 100 µL/well 1 M sulfuric acid. The optical density (OD) of the reaction product was recorded at 405 nm with a microplate reader (Spectra MAX plus, Molecular Devices, Bucher Biotech AG, Switzerland).

### Example 22

Preparation of an influenza vaccine formulation containing DC-Chol : Three bulks of influenza virosomes were prepared by the method described previously (Bron et al., Methods Enzymol. 220:313-331, 1993; Zurbriggen et al., Prog. Lipid Res. 39(1):3-18, 2000). Briefly, 32 mg (41.7 µmol) egg PC and 0.3 - 5 mg (0.6-10 µmol) DC-Chol were dissolved in 2 ml of PBS, 100 mM OEG (OEG-PBS). 4 mg HA of influenza virus (1^{st} bulk A/New Caledonia/20/99 (H1N1); 2^{nd} bulk A/Fujian/411/2002 (H3N2), 3^{ra} bulk B/Shanghai/361/2002) was centrifuged at 100,000 x g for 1 h at 4°C and the pellet was dissolved in 1 ml of PBS/OEG. The detergent solubilized phospholipids and viruses and 1 ml of 20% (w/v) sucrose were mixed to a final volume of 4 ml and sonicated for 1 min. This mixture was centrifuged at 100,000 x g for 1 h at 20°C and the supernatant was sterile filtered (0.22 µm). The three different virosomal bulks were then formed by detergent removal using 180 mg of wet SM2 Bio-Beads for 1 h at room temperature with shaking and three times for 30 min with 90 mg of SM2 Bio-Beads each. The final concentrations of lipids were 8 mg/ml (10.4 µmol/ml) PC, 2 mg/ml (2.7 µmol/ml) PE and 0.075-1.25 mg/ml (0.12-2.5 µmol/ml) DC-Chol.

After HA-quantification the three bulks were mixed and lyophilized. Lyophilization was done in a Savant AES1010 speedvac according to the supplier's instructions. Dried samples were used immediately or stored at -70°C. For reconstitution of lyophilized influenza vaccine formulation, a volume of water equal to the volume before lyophilization was added to the dried DIRIV. Reconstituted empty DIRIV were stored at 4°C.

## Claims

1. A biologically active composition comprising at least one virosome and a cationic lipid for effective lyophilisation and reconstitution of the virosome.

2. A composition according to claim 1, wherein said cationic lipid for effective lyophilisation and reconstitution of the virosome is present in the membrane of the virosome.

3. A composition according to claim 1 or 2, wherein said cationic lipid is a cholesteryl derivative.

4. A composition according to claim 3, wherein said cholesterol derivative has a positively charged substituent in the 3-position of the cholesterol and is represented by the following formula: 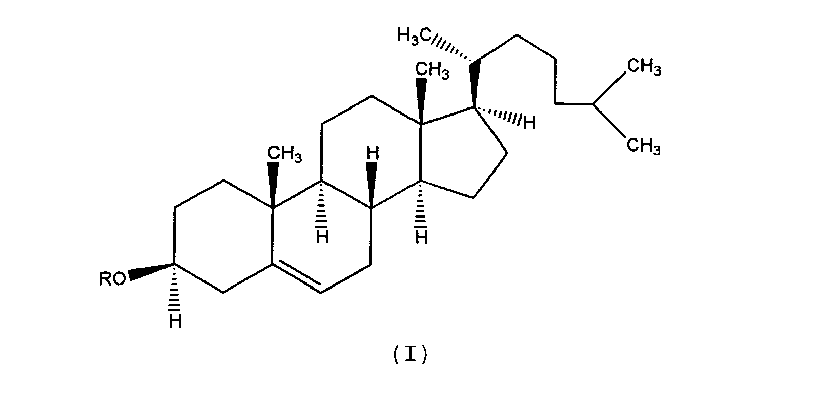 wherein R is selected from the group consisting of R'; R'-(C=O)-; R'-O-(C=O)-; R'-NH-(C=O)-; R'-O-(C=O)-R''-(C=O)-; R'-NH-(C=O)-R''-(C=O)-,
wherein R' is C₁-C₆-alkyl being substituted by at least one positively charged group, preferably a N-containing group of the formula R₁R₂R₃ N⁺- and the respective counter ion is X⁻;
wherein R₁, R₂ and R₃ are independently from each other selected from the group consisting of hydrogen and C₁-C₆₋alkyl;
wherein X⁻ is selected from the group consisting of halogen, hydrogen sulphate, sulfonate, dihydrogen phosphate, acetate, trihaloacetate and hydrogen carbonate; and
wherein R'' is C₁-C₆-alkylene.

5. A composition according to claim 4, wherein said cholesterol derivative is represented by the following formula: 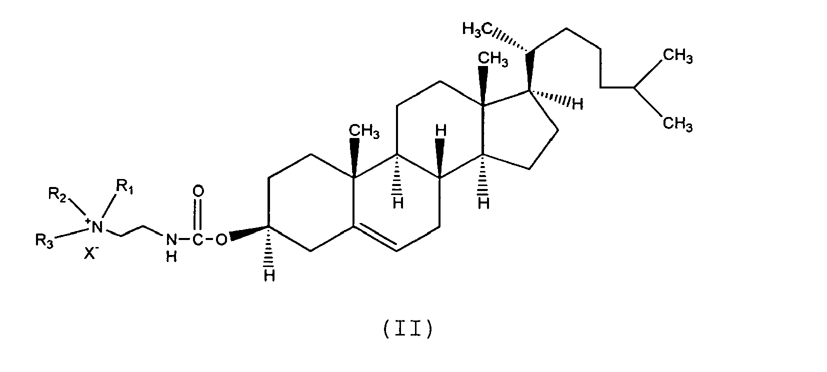 wherein R₁, R₂ and R₃ are independently from each other selected from the group consisting of hydrogen and C₁-C₆₋alkyl, and
wherein X⁻ is a halogen anion.

6. A composition according to claim 5, wherein R₁ and R₂ are methyl and R₃ is hydrogen.

7. A composition according to claim 5, wherein R₁, R₂ and R₃ are methyl.

8. A composition of any of claims 3 to 7, wherein the content of the cationic lipid is between 1.9 and 37 mol% of the total lipid content of the membrane.

9. A composition of claim 8, wherein the content of the cationic lipid is between 1.9 and 16 mol% of the total lipid content of the membrane.

10. A composition according to claim 8 or 9, wherein the residual lipid content of the virosomal membrane consists of phospholipids.

11. A composition according to claim 10, wherein the phospholipids are phosphatidylcholine and phosphatidylethanolamine.

12. A composition according to claim 11, wherein phosphatidylcholine and phosphatidylethanolamine are present in a ratio of 3:1 to 5:1, preferably 4:1.

13. A lyophilization composition comprising the biologically active composition of any of claims 1 to 12, and additionally a lyoprotectant.

14. A composition according to claim 13, wherein the lyoprotectant is selected from the group consisting of sucrose, trehalose, dextrose, lactose, mannose, xylose and mannitol.

15. A composition according to claim 14, wherein the lyoprotectant is present in a ratio of 0.1 to 5%(w/v) in the solution prior to lyophilization.

16. A composition of any of claims 13 to 15, additionally comprising an adjuvant or adjuvant system.

17. A composition of any of claims 1 to 12 or 13 to 16, additionally comprising a biologically active substance selected from a pharmaceutical agent and an antigenic molecule.

18. A composition of claim 17, wherein said biologically active substance is either attached to the surface of the virosome and/or enclosed in the virosome.

19. A method for the lyophilisation of virosomes utilizing the compositions of any of claims 13 to 16 including the steps of:
(a) freezing said composition,
(b) primary drying said frozen composition at a first reduced pressure, and
(c) secondary drying said frozen composition at a second reduced pressure,
wherein said primary drying is carried out at a higher pressure than said second reduced pressure.

20. A method for the lyophilisation of virosomes utilizing the compositions of any of claims 17 or 18 including the steps of:
(a) freezing said composition comprising said biologically active substance selected from a pharmaceutical agent and an antigenic molecule,
(b) primary drying said frozen composition at a first reduced pressure, and
(c) secondary drying said frozen composition at a second reduced pressure,
wherein said primary drying is carried out at a higher pressure than said second reduced pressure.

21. A virosome lyophilisate obtainable by the method of claim 19.

22. A virosome lyophilisate obtainable by the method of claim 20.

23. A method for the reconstitution of a virosome lyophilisate of claim 21 including the step of solubilizing the virosome lyophilisate in a reconstitution solvent.

24. A method according to claim 23, wherein the reconstitution solvent comprises said biologically active substance selected from a pharmaceutical agent and an antigenic molecule.

25. A method for the reconstitution of a virosome lyophilisate of claim 22 including the step of solubilizing the virosome lyophilisate in a reconstitution solvent.

26. The use of any of the compositions of claims 1 to 18 for the manufacture of a pharmaceutical for inoculating a subject therewith.

27. The use of claim 26, wherein the subject is a human.

28. A kit comprising a container containing the lyophilisate of claim 21.

29. A kit according to claim 28, further comprising second container containing a reconstitution solvent and said biologically active substance selected from a pharmaceutical agent and an antigenic molecule.

30. A kit comprising a container containing the lyophilisate of claim 22.

31. A kit according to claim 30, further comprising second container containing a reconstitution solvent.,

32. Use of a cationic cholesteryl derivative for enhancing the immunogenicity of a virosome.

33. Use of a cationic cholesteryl derivative for enhancing the reconstitutability of a lyophilized virosome, said virosome comprising, in its reconstituted state, a biologically active substance selected from a pharmaceutical agent and an antigenic molecule.

34. The use according to claim 32 or 33, wherein said cholesterol derivative has a positively charged substituent in the 3-position of the cholesterol and is represented by the following formula: 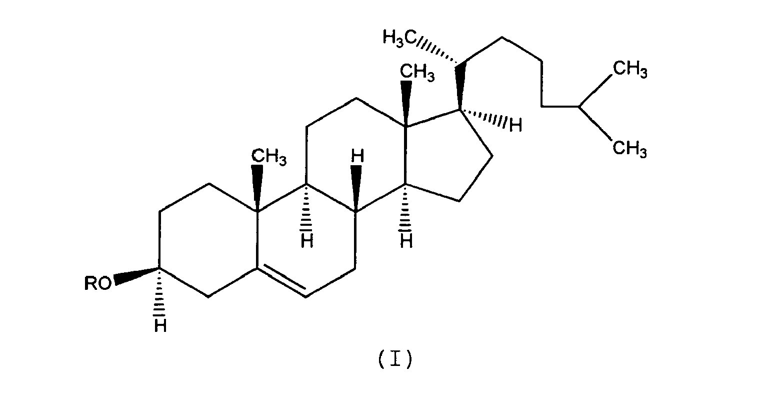 wherein R is selected from the group consisting of R'; R'-(C=O)-; R'-O-(C=O)-; R'-NH-(C=O)-; R'-O-(C=O)-R''-(C=O)-; R'-NH-(C=O)-R''-(C=O)-,
wherein R' is C₁-C₆-alkyl being substituted by at least one positively charged group, preferably a N-containing group of the formula R₁R₂R₃ N⁺- and the respective counter ion is X⁻;
wherein R₁, R₂ and R₃ are independently from each other selected from the group consisting of hydrogen and C₁-C₆₋alkyl;
wherein X⁻ is selected from the group consisting of halogen, hydrogen sulphate, sulfonate, dihydrogen phosphate, acetate, trihaloacetate and hydrogen carbonate; and
wherein R'' is C₁-C₆-alkylene.

35. The use according to claim 34, wherein said cholesterol derivative is represented by the following formula: 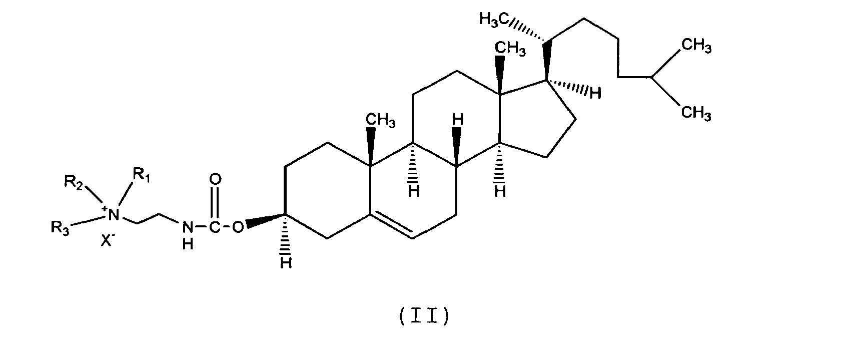 wherein R₁, R₂ and R₃ are independently from each other selected from the group consisting of hydrogen and C₁-C₆₋alkyl, and
wherein X⁻ is a halogen anion.

36. The use according to claim 35, wherein R₁ and R₂ are methyl and R₃ is hydrogen.

37. The use according to claim 35, wherein R₁, R₂ and R₃ are methyl.

38. The use of any of claims 32 to 37, wherein the content of the cationic lipid in the virosomal membrane is between 1.9 and 37 mol% of the total lipid content of the membrane.

39. The use of claim 38, wherein the content of the cationic lipid in the virosomal membrane is between 1.9 and 16 mol% of the total lipid content of the membrane.

40. The use according to claim 38 or 39, wherein the residual lipid content of the virosomal membrane consists of phospholipids.

41. The use according to claim 40, wherein the phospholipids are phosphatidylcholine and phosphatidylethanolamine.

42. The use according to claim 41, wherein phosphatidylcholine and phosphatidylethanolamine are present in a ratio of 3:1 to 5:1, preferably 4:1.

43. The use of any of claims 32 to 42, additionally comprising an adjuvant or adjuvant system.
